# FASCICULE DE BREVET EUROPEEN

(11) **EP 4 360 554 B1**
(45) Date de publication et mention de la délivrance du brevet: **16.04.2025**
(21) Numéro de dépôt: 23202178.2
(22) Date de dépôt: 06.10.2023
(51) Int. Cl.: A61B 5/293, A61B 5/00

(54) **SYSTÈME DE MESURE D'UNE ACTIVITÉ NEURONALE À DISPOSITIF DE FIXATION AMÉLIORÉ**
SYSTEM ZUR MESSUNG NEURONALER AKTIVITÄT MIT VERBESSERTER BEFESTIGUNGSVORRICHTUNG
SYSTEM FOR MEASURING NEURAL ACTIVITY WITH IMPROVED FIXATION DEVICE

(30) Priorité: 26.10.2022 FR 2211128
(43) Date de publication de la demande: 01.05.2024
(73) Titulaire: Commissariat à l'Energie Atomique et aux Energies Alternatives, 75015 Paris (FR)
(72) Inventeur: SAUTER-STARACE, Fabien, 38054 Grenoble cedex 09 (FR); CHARVET, Guillaume, 38054 Grenoble cedex 09 (FR)
(74) Mandataire: INNOV-GROUP

(56) Documents cités:
- EP-B1- 2 623 025
- US-B1- 7 346 391
- US-B1- 9 622 677

## Description

### Domaine technique de l'invention

La présente invention se rapporte à un système de mesure de l'activité neuronale d'un être vivant, le système étant doté d'un dispositif de fixation amélioré.

### Etat de la technique

Il existe de nombreux dispositifs implantables dans le cerveau d'un être vivant, utilisés pour la lecture, l'enregistrement et/ou le contrôle de signaux neuronaux.

Les documents brevets référencés US7346391 B1, US9622677B1, EP2623025B1, WO2011/123150A1.

WO2011/067297A1 et US7747318B2 décrivent différents dispositifs de mesure de l'activité neuronale d'un être vivant.

Ces dispositifs comportent en général un boîtier venant se loger partiellement ou en totalité dans une cavité (après une craniotomie) réalisée sur le crâne. Il comporte également un système d'électrodes de mesure de l'activité neuronale, agencé sur une face du boîtier, et des moyens électroniques de traitement de signaux logés dans le boîtier. Il comporte également au moins une antenne couplée aux moyens électroniques pour envoyer des données mesurées vers un terminal distant.

Dans certains cas, le dispositif peut être parfaitement intégré et suivre la forme du crâne (comme dans le brevet EP2623025B1), ou comporter une partie logée dans le crâne et une partie saillante par rapport à la surface du crâne (comme dans la demande de brevet WO2011/123150A1).

Ce type de dispositif de mesure doit répondre à plusieurs contraintes :
- Il doit être parfaitement positionné et ne pas bouger dans sa cavité, quelle que soit l'activité du patient ;
- Il doit également être positionné de sorte que son système d'électrodes vienne le mieux possible en contact avec le cerveau pour optimiser la prise de mesure ;
- Il doit permettre d'éviter toute lésion, notamment en cas de choc ;
- Il doit pouvoir être facilement localisé, même après cicatrisation ;

Le but de l'invention est de proposer un système de mesure permettant de répondre à une ou plusieurs des contraintes évoquées ci-dessus.

### Exposé de l'invention

Ce but est atteint par un système de mesure de l'activité neuronale d'un être vivant, selon la revendication 1.

Selon une première réalisation, le dispositif de maintien mécanique comporte trois organes de bridage, chaque organe de bridage étant destiné à être vissé dans la boîte crânienne par lesdits moyens de fixation et agencé pour venir en appui contre une ailette du boîtier.

Selon une particularité, les moyens de réglage comportent une rondelle destinée à être placée entre l'ailette du boîtier et une surface d'appui située sur le pourtour de la cavité, l'épaisseur de la rondelle définissant la profondeur d'enfoncement du boîtier dans la cavité.

Selon une autre particularité, chaque organe de bridage est réalisé dans un matériau amagnétique et non conducteur électrique.

Selon une deuxième réalisation non couverte par les revendications, le dispositif de maintien mécanique comporte une pièce d'accueil destinée à venir se loger dans la cavité, fixée sur le pourtour de la cavité par lesdits moyens de fixation, ladite pièce d'accueil portant lesdits moyens de réglage.

Selon une particularité, les moyens de réglage comportent un filetage interne réalisé sur une surface de la pièce d'accueil et configuré pour coopérer avec un filetage externe réalisé sur une surface du boîtier.

Selon une autre particularité, le système comporte des moyens de blocage du boîtier en profondeur dans la pièce d'accueil.

Selon une autre particularité, la pièce d'accueil comporte un ou plusieurs logements, destinés chacun à recevoir, de manière amovible, au moins un aimant permanent. Selon une autre particularité, le boîtier et/ou la pièce d'accueil comporte des moyens mécaniques d'aide au vissage du boîtier dans la pièce d'accueil.

Selon une autre particularité, la pièce d'accueil est réalisée dans un matériau amagnétique et non conducteur électrique.

Selon une troisième réalisation pas couvert par le texte des revendications, le dispositif de maintien mécanique comporte plusieurs organes de bridage répartis autour du boîtier du dispositif de mesure, chaque organe de bridage comportant un corps destiné à être fixé dans la boîte crânienne et une plaquette réglable en hauteur par rapport au corps et enserrant le boîtier par son épaisseur. Selon une particularité, les moyens de réglage en hauteur comportent une vis sans fin coopérant avec ladite plaquette et avec le corps de l'organe de bridage.

### Brève description des figures

D'autres caractéristiques et avantages vont apparaître dans la description détaillée qui suit faite en regard des dessins annexés dans lesquels :
- Les figures 1A et 1B représentent une première variante de réalisation du système, respectivement en vue de dessus et en vue suivant une coupe transversale ;
- Les figures 2A et 2B représentent une deuxième variante de réalisation du système pas couverte par le texte des revendications, respectivement en vue de dessus et en vue suivant une coupe transversale ;
- Les figures 3A et 3B représentent une troisième variante de réalisation du système pas couverte par le texte des revendications, respectivement en vue de dessus et en vue suivant une coupe transversale partielle ;

### Description détaillée d'au moins un mode de réalisation

Selon l'invention, le système de mesure comporte les caractéristiques de la revendication 1. Les deuxième et troisième variantes de réalisation ci-dessous ne sont pas couvertes par le texte des revendications.

Le dispositif de mesure 1 comporte un système 11 d'électrodes de mesure de l'activité neuronale, agencé sur une face inférieure du boîtier 10, ladite face inférieure étant destinée à être orienté vers le fond de ladite cavité lorsque le boîtier est en place. Les électrodes 110 sont chacune destinées à venir en contact avec les tissus 4 dont l'activité électrique est à mesurer.

Il comporte également des moyens 12 électroniques de traitement de signaux (notamment filtrage, amplification, numérisation des signaux) logés dans le boîtier 10, sur lequel est connecté le système 11 d'électrodes. Il peut également comporter une antenne A1 de transmission de données, connectée aux moyens électroniques de traitement pour transférer les données numérisées vers un terminal externe distant, et une antenne A2 de télé-alimentation utilisée pour apporter l'énergie à la partie électronique et/ou pour charger une batterie du dispositif de mesure 1 par couplage inductif. La batterie peut être logée dans le boîtier du dispositif de mesure.

Les deux antennes A1, A2 peuvent être réalisées sur un support 13 fixé audit boîtier ou intégrées autour ou dans le boitier. En particulier, l'antenne A1 de télé-alimentation peut être placée dans la circonférence du boitier a fortiori si celui-ci est en titane (qui est un matériau qui pénalise l'alimentation par couplage inductif, en particulier à 13.56MHz). L'antenne A2 peut être envisagée en périphérie dans une couronne du boîtier, agencée sur sa face supérieure ou dans le boitier 10 en fonction des fréquences de télécommunication et des performances attendues.

Selon l'invention, le dispositif de fixation 2, 3, 4 du système est agencé pour tenir le boîtier 10 dans la cavité réalisée dans la boîte crânienne C et permettre de maintenir les électrodes 110 en appui contre les tissus 4.

Le dispositif de fixation 2, 3, 4 comporte au moins un dispositif de maintien mécanique, chargé de maintenir le boîtier 10 dans sa cavité.

Le dispositif de fixation 2, 3, 4 comporte également des moyens de fixation arrangés pour fixer le dispositif de maintien mécanique dans la boîte crânienne C, sur le pourtour de la cavité, pour maintenir le boîtier 10 dans la cavité.

Ces moyens de fixation peuvent comporter avantageusement plusieurs vis 20, 30, 40 de type auto-foreuses traversant le dispositif de maintien en plusieurs points et destinés à venir se visser dans la boîte crânienne C.

Selon l'invention, le dispositif de fixation comporte également des moyens de réglage de la position en profondeur du boîtier 10 dans la cavité. Ces moyens de réglage sont destinés à positionner le boîtier 10 de manière optimale pour que son système 11 d'électrodes vienne en contact mécanique avec les tissus 4, pour une prise de mesure fiable, sans exercer une contrainte trop prononcée.

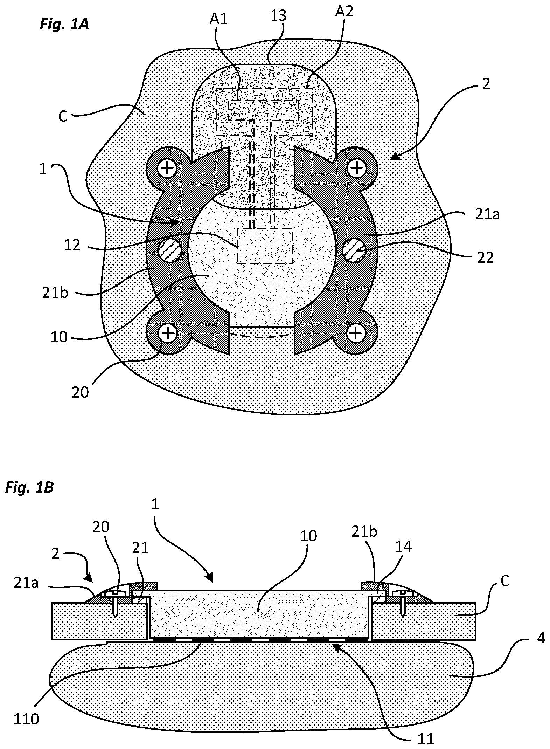

Dans une première variante de réalisation illustrée par la figure 1A et la figure 1B, le dispositif de maintien mécanique du dispositif de fixation 2 comporte un ou plusieurs organes de bridage 21. Sur une variation, pas couverte par le texte des revendications, de première variante, deux organes de bridage 21a, 21b sont par exemple utilisés. Les deux organes de bridage 21a, 21b sont chacun réalisés sous la forme d'une pièce en demi-lune venant se visser dans la boîte crânienne C et prendre appui sur une ou plusieurs ailettes 14 latérales du boîtier 10 du dispositif de mesure. Les ailettes 14 latérales peuvent être remplacées par une collerette réalisée tout autour du boîtier 10. Les ailettes 14 ou la collerette sont agencées de manière à venir se positionner en vis-à-vis de la surface de la boîte crânienne C, cette surface étant présente sur le pourtour de la cavité.

Les moyens de réglage de la position en profondeur du boîtier 10 comportent avantageusement une rondelle 21 ou équivalent, positionnée à l'interface entre les ailettes 14 (ou la collerette) du boîtier 10 et la surface de la boîte crânienne C située sur le pourtour de la cavité. L'épaisseur de la rondelle 21 permet de régler la position en profondeur du boîtier 10 dans la cavité. La rondelle 21 peut être remplacée par toute autre solution équivalente, telle que joint en matériau souple de type silicone ou polycarbonate implantable, ou rigide tel que, de manière non exhaustive, du téflon (marque déposée), PEEK, PEKK ou du ciment dentaire, etc. Bien entendu, selon l'épaisseur de la rondelle 21, la profondeur de vissage des organes de bridage 21a, 21b sera adaptée. Le matériau employé pour cette "couche" de réglage sera bien entendu choisi biocompatible.

Selon l'invention, chaque organe de bridage 21a, 21b présente un logement dans lequel est placé, de manière amovible, un aimant permanent 22. Les aimants permanent 22 permettent de mieux localiser le dispositif une fois implanté, notamment son antenne de télé-alimentation A2 pour alimenter le dispositif ou recharger sa batterie. Grâce aux aimants permanents, il est possible de le localiser en position (suivant X et Y) mais aussi en profondeur, en tenant compte de l'intensité du champ magnétique généré. Par ailleurs, l'utilisation de trois aimants permanents (organisés en triangle) permet de localiser le boîtier avec précision (par triangulation).

De manière non limitative, le dispositif de maintien formé de chaque organe de bridage peut être réalisé en titane. Alternativement, pour la compatibilité IRM, il peut également s'agir d'un polymère biocompatible et isolant de type PEEK ou PEKK.

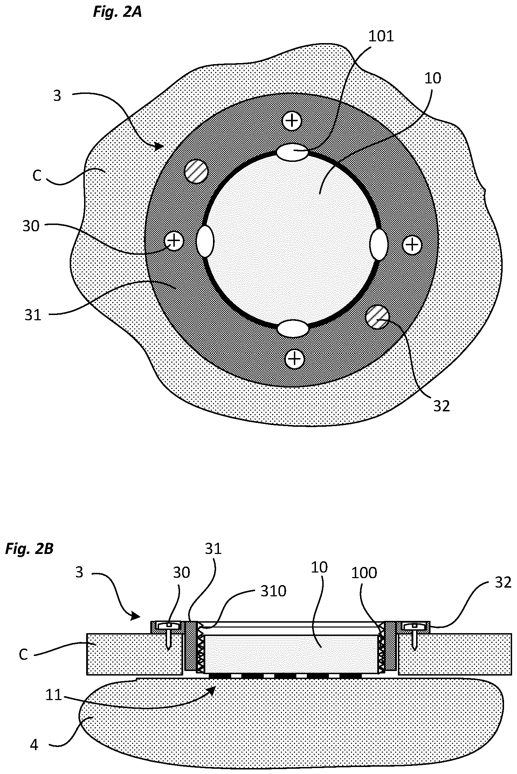

Dans une deuxième variante de réalisation représentée sur la figure 2A et sur la figure 2B, le dispositif de maintien mécanique du dispositif de fixation 3 comporte une pièce d'accueil 31 venant se loger dans la cavité. Cette pièce d'accueil 31 comporte des ailettes latérales 32 ou une collerette venant en appui contre la surface de la boîte crânienne C (table externe) située sur le pourtour de la cavité ou sur un lamage. Les moyens de fixation sont également formés de vis 30 auto-foreuses traversant lesdites ailettes 32 ou la collerette et s'enfonçant dans la boîte crânienne C pour fixer la pièce d'accueil 31. Les vis sont réparties sur le pourtour de la pièce d'accueil 31 pour obtenir une fixation stable et uniforme de la pièce d'accueil 31.

La pièce d'accueil 31 est réalisée sous la forme d'une pièce tubulaire ouverte sur le dessus et sur le dessous. Elle comporte une section interne cylindrique. Sur sa face latérale interne, la pièce d'accueil dispose d'un filetage 310 destiné à coopérer avec un filetage 100 réalisé sur une face latérale du boîtier 10 du dispositif de mesure. Le vissage du boîtier 10 dans la pièce d'accueil 31 permet de régler la position en profondeur du dispositif de mesure 1 et ainsi le niveau d'appui de son système 11 d'électrodes situé sur sa face inférieure contre les tissus 4. L'angle de filetage est préférentiellement adapté au coefficient de frottement des matériaux en conditions humides pour obtenir un filetage irréversible.

Dans cette réalisation, pour assurer le maintien angulaire de l'implant 10 pendant le vissage de la pièce d'accueil 31 ou le vissage du boîtier 10 alors que la pièce d'accueil 31 est calée dans la craniotomie et fixée angulairement au moyen des vis auto-foreuses, différents moyens peuvent être envisagés :
- Des encoches 101 peuvent être réalisées dans la circonférence du boitier 10 au niveau du filetage (figure 2A - celui-ci pouvant comporter des discontinuités). Ces encoches sont préférentiellement en périphérie et traversantes pour simplifier la qualification du nettoyage de l'implant et éviter des complications chirurgicales liées aux frictions sur des aspérités ou arêtes en cas de contact avec la peau ;
- Une languette peut être collée avec un adhésif silicone biocompatible offrant une faible adhérence (sans promoteur d'adhérence) ;
- Une ventouse peut être appliquée temporairement sur la face supérieure du boîtier 10 pour le maintenir ou l'actionner en rotation par rapport à la pièce d'accueil ;

Des moyens de blocage peuvent être prévus pour bloquer la position en profondeur du boîtier 10 vissé dans la pièce d'accueil 31. Ces moyens de blocage peuvent être réalisés par différentes solutions, par exemple via une encoche et une barre de verrouillage, ou à l'aide d'un clip anti-retour venant se positionner dans le filetage pour entraver le déplacement du boîtier.

La pièce d'accueil 31 est réalisée dans un matériau biocompatible, facile à usiner, amagnétique et isolant tel que le PEEK ou le PEKK. Le filetage 100 réalisé sur le boîtier 10 du dispositif de mesure peut être obtenu à partir d'un moulage ou rapporté par collage. Le matériau préconisé est également biocompatible et amagnétique.

De même, la pièce d'accueil 31 peut comporter un ou plusieurs logements destinés chacun à recevoir un aimant permanent 32 permettant la localisation du boîtier 10 et celle de son antenne de télé-alimentation A2.

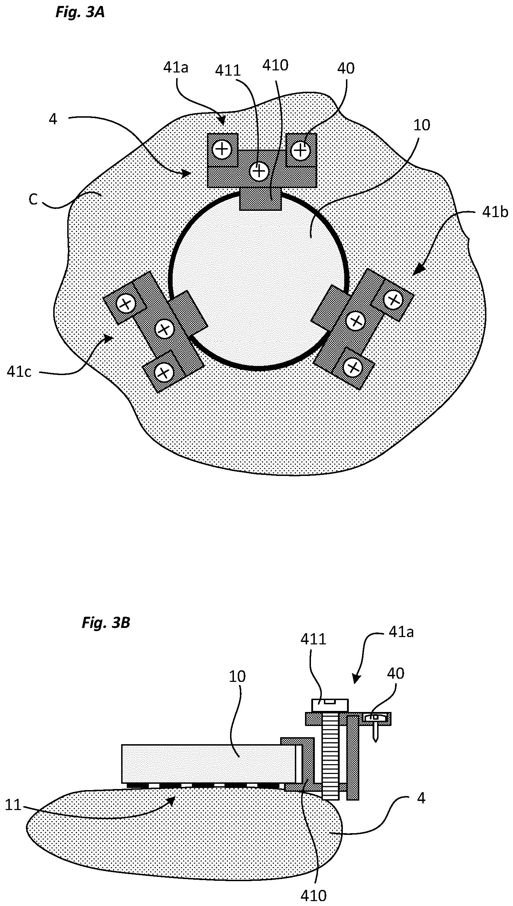

Dans une troisième variante de réalisation illustrée par la figure 3A et la figure 3B, le dispositif de maintien mécanique du dispositif de fixation 4 comporte au moins trois organes de bridage 41a, 41b, 41c, avantageusement identiques, séparées entre eux et réparties autour du boîtier 10 du dispositif de mesure 1. Les trois organes de bridage 41 sont donc avantageusement disposés à 120° les uns des autres.

De manière avantageuse, chaque organe de bridage 41a, 41b, 41c peut être fixé dans la boîte crânienne C via une ou plusieurs vis 40 (par exemple de type auto-foreuse). Chaque organe de bridage 41a, 41b, 41c comporte également une plaquette 410 réglable en hauteur et configurée pour coopérer avec le boîtier 10 du dispositif. Cette plaquette 410 peut présenter une section en U, lui permettant d'enserrer le boîtier 10 sur son épaisseur. Le réglage en hauteur de la plaquette 410 peut être réalisé grâce à une vis sans fin 411 insérée dans le corps de l'organe de bridage. Cette solution de réglage permet d'obtenir un réglage fin de la position du dispositif de mesure 1 (selon trois axes) et d'orienter le boitier pour pouvoir mieux tenir compte de la forme des tissus 4, avec lesquels le système d'électrodes 11 est amené à venir en contact. De manière avantageuse, l'interface avec le boitier sera plutôt de type ponctuelle afin de ne pas contraindre l'orientation des surfaces. L'orientation du plan du système d'électrodes 11 est réglée au moyen des trois vis sans-fin 411, à l'instar d'un trépied.

Cette version à trois organes de bridage est avantageuse en ce que chacun des organes peut porter un aimant permanent distinct, facilitant la localisation du boîtier 10 et de son antenne de télé-alimentation A2, par triangulation.

La solution de l'invention présente de nombreux avantages, parmi lesquels :
- Elle permet un maintien fiable du dispositif de mesure dans sa cavité ;
- Elle permet un réglage en profondeur de la position du boîtier du dispositif de mesure, garantissant le contact du système d'électrodes contre les tissus, sans risque de lésions ;
- Elle est adaptée au dispositif de mesure existant, tel que celui décrit dans le brevet EP2623025B1 ;

## Revendications

1. Système de mesure de l'activité neuronale d'un être vivant, comprenant :
- Un dispositif de mesure (1) implantable doté d'un boîtier (10) destiné à être logé, au moins partiellement, dans une cavité formée dans la boîte crânienne (C) d'un être vivant, et comprenant un système (11)d'électrodes de mesure de l'activité neuronale agencé sur une face du boîtier (10), et des moyens (12) électroniques de traitement de signaux logés dans le boîtier, auxquels est connecté le système (11)d'électrodes,
- Un dispositif de fixation (2, 3) du boîtier dans ladite cavité,
le dispositif de fixation (2, 3) comportant :
- Au moins un dispositif de maintien mécanique dudit boîtier (10) dans la cavité,
- Des moyens de fixation dudit dispositif de maintien mécanique, agencés pour fixer le dispositif de maintien dans la boîte crânienne (C) sur le pourtour de la cavité,
- Des moyens de réglage de la position en profondeur du boîtier (10) dans la cavité,
- Des moyens de localisation dudit boîtier (10), lesdits moyens de localisation comportant un ou plusieurs aimants permanents,
- Le système étant **caractérisé en ce que** ledit dispositif de maintien mécanique comporte trois organes de bridage,
- Chaque organe de bridage comportant un ou plusieurs logements, destinés chacun à recevoir, de manière amovible, au moins un aimant permanent (22) desdits moyens de localisation.

2. Système de mesure selon la revendication 1, **caractérisé en ce que** chaque organe de bridage (21a, 21b) est destiné à être vissé dans la boîte crânienne (C) par lesdits moyens de fixation et agencé pour venir en appui contre une ailette (14) du boîtier (10).

3. Système de mesure selon la revendication 2, **caractérisé en ce que** les moyens de réglage comportent une rondelle (21) destinée à être placée entre l'ailette (14) du boîtier et une surface d'appui située sur le pourtour de la cavité, l'épaisseur de la rondelle (21) définissant la profondeur d'enfoncement du boîtier dans la cavité.

4. Système de mesure selon l'une des revendications 1 à 3, **caractérisé en ce que** chaque organe de bridage (21a, 21b) est réalisé dans un matériau amagnétique et non conducteur électrique.

## Patentansprüche

1. System zur Messung der neuronalen Aktivität eines Lebewesens, beinhaltend:
- eine implantierbare Messvorrichtung (1), die über ein Gehäuse (10) verfügt, das dazu bestimmt ist, mindestens teilweise in einer Höhle aufgenommen zu werden, die im Schädel (C) eines Lebewesens gebildet ist, und die ein Elektrodensystem (11) zur Messung der neuronalen Aktivität, das an einer Seite des Gehäuses (10) eingerichtet ist, und elektronische Signalverarbeitungsmittel (12), die in dem Gehäuse aufgenommen sind und mit denen das Elektrodensystem (11) verbunden ist, beinhaltet,
- eine Vorrichtung zur Befestigung (2, 3) des Gehäuses in der Höhle,
wobei die Befestigungsvorrichtung (2, 3) Folgendes umfasst:
- mindestens eine Vorrichtung zum mechanischen Halten des Gehäuses (10) in der Höhle,
- Mittel zum Befestigen der mechanischen Haltevorrichtung, die dazu eingerichtet sind, die Haltevorrichtung in dem Schädel (C) an dem Umfang der Höhle zu befestigen,
- Mittel zum Einstellen der Tiefenposition des Gehäuses (10) in der Höhle,
- Mittel zum Lokalisieren des Gehäuses (10), wobei die Lokalisierungsmittel einen oder mehrere Permanentmagneten umfassen,
- wobei das System **dadurch gekennzeichnet ist, dass** die mechanische Haltevorrichtung drei Klemmelemente umfasst,
- wobei jedes Klemmelement eine oder mehrere Aufnahmen umfasst, die jeweils dazu bestimmt sind, mindestens einen Permanentmagneten (22) der Lokalisierungsmittel entfernbar aufzunehmen.

2. Messsystem nach Anspruch 1, **dadurch gekennzeichnet, dass** jedes Klemmelement (21a, 21b) dazu bestimmt ist, mit Hilfe der Befestigungsmittel in den Schädel (C) geschraubt zu werden, und dazu eingerichtet ist, an einem Flügel (14) des Gehäuses (10) zur Anlage zu kommen.

3. Messsystem nach Anspruch 2, **dadurch gekennzeichnet, dass** die Einstellmittel eine Scheibe (21) umfassen, die dazu bestimmt ist, zwischen dem Flügel (14) des Gehäuses und einer Anlagefläche, die sich am Umfang der Höhle befindet, platziert zu werden, wobei die Dicke der Scheibe (21) die Eindringtiefe des Gehäuses in die Höhle definiert.

4. Messsystem nach einem der Ansprüche 1 bis 3, **dadurch gekennzeichnet, dass** jedes Klemmelement(21a, 21b) aus einem unmagnetischen und elektrisch nicht leitfähigen Material hergestellt ist.

## Claims

1. System for measuring the neural activity of a living being, comprising:
- an implantable measuring device (1) equipped with a housing (10) to be accommodated, at least partially, in a cavity formed in the cranium (C) of a living being, and comprising a system (11) of electrodes for measuring the neural activity, arranged on one face of the housing (10), and electronic signal-processing means (12) which are accommodated in the housing and to which the electrode system (11) is connected,
- a device (2, 3) for fixing the housing in said cavity,
the fixing device (2, 3) comprising:
- at least one device for mechanically holding said housing (10) in the cavity,
- means for fixing said mechanical holding device, which are arranged to fix the holding device in the cranium (C) on the periphery of the cavity,
- means for adjusting the depth position of the housing (10) in the cavity,
- means for locating said housing (10), said locating means comprising one or more permanent magnets,
- the system being **characterized in that** said mechanical holding device comprises three clamping members,
- each clamping member comprising one or more housings, each intended to receive, in a removable manner, at least one permanent magnet (22) of said locating means.

2. Measuring system according to Claim 1, **characterized in that** each clamping member (21a, 21b) is intended to be screwed into the cranium (C) by said fixing means and arranged to bear against a wing (14) of the housing (10).

3. Measuring system according to Claim 2, **characterized in that** the adjustment means comprise a washer (21) intended to be placed between the wing (14) of the housing and a bearing surface situated on the periphery of the cavity, the thickness of the washer (21) defining the depth of engagement of the housing in the cavity.

4. Measuring system according to one of Claims 1 to 3, **characterized in that** each clamping member (21a, 21b) is made of a non-magnetic and electrically non-conductive material.
